# EUROPÄISCHE PATENTSCHRIFT

(11) **EP 1 065 987 B1**
(45) Veröffentlichungstag und Bekanntmachung des Hinweises auf die Patenterteilung: **17.12.2003**
(21) Anmeldenummer: 99917855.1
(22) Anmeldetag: 25.03.1999
(51) Int. Cl.: A61B 19/00

(54) **CAS-ERFASSBARER ENDOSKOP-ADAPTER**
ENDOSCOPE ADAPTER WHICH CAN BE DETECTED USING COMPUTER ASSISTED SURGERY
ADAPTATEUR POUR ENDOSCOPE POUVANT ETRE DETECTE PAR UN SYSTEME DE CHIRURGIE ASSISTEE PAR ORDINATEUR

(30) Priorität: 26.03.1998 DE 19813383
(43) Veröffentlichungstag der Anmeldung: 10.01.2001
(73) Patentinhaber: Karl Storz GmbH & Co. KG, 78532 Tuttlingen (DE)
(72) Erfinder: IRION, Klaus, M., D-78576 Liptingen (DE); REBHOLZ, Clemens, 88690 Uhldingen-Mühlhofen (DE)
(74) Vertreter: Weller, Wolfgang, Dr.
(86) Internationale Anmeldenummer: EP9902021
(87) Internationale Veröffentlichungsnummer: WO99048432

(56) Entgegenhaltungen:
- EP-A- 0 672 389
- US-A- 4 478 212
- US-A- 5 517 990
- US-A- 5 617 857

## Beschreibung

Die Erfindung betrifft einen Adapter, zum Verbinden zumindest zweier medizinischer Instrumente, mit einer Anschlußkupplung an einer ersten Seite, über die der Adapter an eine vorbestimmte Koppelstelle an dem einen medizinischen Instrument lösbar, jedoch mechanisch festsitzend einfach ankoppelbar ist, und wobei der Adapter zumindest eine weitere Anschlußkupplung aufweist, über die er mit einem weiteren medizinischen Instrument koppelbar ist.

Ein derartiger Adapter ist aus dem Dokument US-A-4 478 212 bekannt.

Aus den Druckschriften US-A-5 517 990 und US-A-5 617 857 sind Stäbe bzw. Handstücke bekannt, die in Verbindung zu medizinischen Instrumenten stehen, wobei die Stäbe bzw. Handstücke Sendereinheiten aufweisen.

Aus Cinquin, P. et al.: "Computer Assisted Medical Intervention", IEEE Engineering in Medicine and Biology, Mai/Juni 1995, Seiten 256 bis 263 ist eine Vorrichtung mit einer Sendereinheit, über die die Position eines medizinischen Instrumentes mit Hilfe eines CAS-Systems erfaßbar ist, bekannt.

Bei verschiedenen Operationen, wie z.B. in der Neurochirurgie, in der wirbelsäulenchirurgie und in der Chirurgie im HNO-Bereich, werden zunehmend sogenannte CAS-Systeme (Computer Assisted Surgery) eingesetzt. Derartige Systeme werden auch mit dem Kürzel CAMI = Computer Assisted Medical Interventions bezeichnet. Diese CAS-Systeme bestehen aus mindestens einer Positionssensoreinheit, die die Lage und/oder die Richtung eines Instrumentes, bspw. eines Endoskops, erfaßt und diese Information an eine Bildverarbeitungseinheit überträgt, in der Daten in Form von CT-, MR- und/oder Ultraschallbildern über den zu therapierenden Körperbereich in dreidimensionaler Form vorliegen. Nachdem über Referenzmarkierungen originale Positionsdaten korrespondierenden dreidimensionalen Bilddaten zugeordnet werden, ist eine lagerichtige Positionserkennung von Instrumenten im Bilddatensatz möglich. Der Operateur besitzt somit die Möglichkeit, sich während einer Operation anhand der auf einem Monitor wiedergegebenen Bilddaten zu orientieren. Voraussetzung für eine möglichst genaue Lokalisierung ist neben der Genauigkeit der Positionserkennung vor allem die geometrische Invarianz der Körperstruktur vom Zeitpunkt der Bilddatenerfassung bis zur Operation. Dieses Verfahren eignet sich daher vor allem für Operationen, bei denen harte Körperstrukturen involviert sind und die Weichgewebsverschiebbarkeit gering ist, also Operationen an der Schädelbasis, an der Wirbelsäule, in der Sinuschirurgie.

Bei der aus Cinquin, P. et al. a.a.O. bekannten Vorrichtung erfolgt die Positionserfassung über mehrere abstrahlende Dioden- bzw. Positionselemente, deren Signale von entfernt liegenden Kameras erfaßt und einem Rechner zugeführt werden. Über Triangulationsverfahren werden im Rechner die Positionen der Dioden und somit die Lage der direkt damit verbundenen Instrumente bestimmt. Die Sendereinheit muß mit dem Instrument, bspw. dem Endoskop, dessen Lage bestimmt werden soll, lagefest verbunden sein. Diese Verbindung muß zumindest für die Dauer der Operation invariant bleiben. Bei der eingangs genannten Vorrichtung sind insgesamt sechs Dioden auf einem etwa T- oder schwertförmigen sperrigen und voluminösen Bauteil enthalten, das im Bereich eines Schaftes an das jeweilige Instrument angebracht ist, nämlich entweder am Schaft eines Endoskopes oder am Schaft eines Bohrers. Eine über den Schaft geschobene Hülse erlaubt es, die Vorrichtung längs des Schaftes zu positionieren und über Feststellschrauben zu fixieren.

Nachteilig bei dieser bekannten Vorrichtung ist, daß die dort durchgeführten Ankopplungen und ein Wechsel der Sendereinheit auf ein anderes Instrument, bspw. ein anderes Endoskop, sehr zeitaufwendig und kompliziert ist.

Ein derartiger Wechsel ist vor allem unter klinischen Bedingungen während einer Operation und unter sterilen Anforderungen nur mit hohem Aufwand durchzuführen.

Bei zahlreichen Operationen werden mehrere Instrumente eingesetzt, und im Laufe einer Operation ist es erforderlich, ein zu Beginn der Operation eingesetztes Instrument, das zwischenzeitlich beiseite gelegt wurde, erneut einzusetzen.

Ist bspw. das Instrument ein Endoskop mit einem sogenannten Geradeausblick, so kann dieses Endoskop zu Beginn der Operation dazu verwendet werden, um bspw. über eine Verschiebung längs der Endoskopachse an einen Operationsort herangeführt zu werden, bspw. einem Ort in der Stirnhöhle über der Nase. Soll nun eine Operation bspw. an einem seitlich an der Operationsstelle gelegenen Tumor durchgeführt werden, wird das Endoskop mit dem Geradeausblick durch ein Endoskop mit einem Seitenblick ersetzt.

Soll am Ende der Operation das Operationsfeld noch einmal auf verbliebene Gegenstände inspiziert werden, ist bspw. wieder das eingängliche Endoskop mit dem Geradeausblick notwendig. Darüber hinaus ist auch häufig der Wechsel von oder zu einem passiven Instrument notwendig, beispielsweise, wenn ein Trokar mit einem Obturator, d.h. ohne Sicht, eingeführt wird.

Damit nun die Position des jeweiligen Instruments bestimmt werden kann, muß jedes Instrument mit zahlreichen, zumindest drei, Sendern versehen werden. Dies ist sehr aufwendig und teuer.

Es sind also während einer Operation zahlreiche Ankoppel- und Lösevorgänge zwischen den Komponenten des medizinischen Instrumentariums notwendig. Die Komponenten, die an ein Endoskop angekoppelt werden, sind meist Kameras, die über Kabel mit der Prozessoreinheit und einem Monitor verbunden sind. Dabei stellt sich das Problem der Kontamination der sterilen Instrumente über die nicht-sterilen Bauelemente, wie Kameras oder dgl.

Es ist Aufgabe der vorliegenden Erfindung, einen Adapter zu schaffen, der in Verbindung mit einem CAS-System zu benutzen ist.

Erfindungsgemäß wird die Aufgabe dadurch gelöst, daß der Adapter eine Sendereinheit aufweist, über die die Position des einen, an den Adapter angekoppelten medizinischen Instrumentes mit Hilfe eines CAS-Systemes erfaßbar ist, wobei die Sendereinheit lagefest zum Adapter ist, und wobei die Sendereinheit mehrere auf der Sendereinheit verteilt angeordnete Sender aufweist, deren Position vom CAS-System erfaßbar ist.

Es ist nicht mehr notwendig, an jedem Instrument eine Sendereinheit vorzusehen, sondern es ist nur an das zu erfassende Instrument der Adapter anzukoppeln, was durch einen einfach durchzuführenden Vorgang zu bewerkstelligen ist. Der Adapter muß nur an die entsprechende, am Instrument vorhandene Koppelstelle angesetzt und gekoppelt werden, was durch eine Art Aufklipsen durchgeführt werden kann. Der Adapter kann an unterschiedlichste Instrumente angekoppelt werden, diese müssen lediglich die entsprechende Koppelstelle für den Adapter aufweisen. Es können nun bei einer Operation verschiedene Instrumente an den Adapter angekoppelt bzw. von diesem gelöst werden, was sehr einfach und ohne hohe Aufmerksamkeit und auch sehr rasch durchzuführen ist. Soll die Position des distalen Endes, also die Spitze des Instrumentes, erfaßt werden, ist beim Wechseln des Instrumentes nur noch die Information notwendig, wieweit die Spitze von dem Adapter entfernt ist, was bspw. durch entsprechende Eingaben und Speicherung in der Prozessoreinheit oder durch entsprechende Instrumentenmerkmale von dem CAS-System selbst erfaßt werden kann.

Das Vorsehen dieses Adapters eröffnet nun auch die Möglichkeit, sterile Bereiche des medizinischen Instrumentariums, bspw. ein Endoskop, von nicht-sterilen Bereichen, bspw. einer Kamera mit den entsprechenden Anschlußkabeln an den im Operationssaal befindlichen Monitoren, an einer definierten Stelle zu trennen. Es braucht nur noch der Adapter mit seiner Sendereinheit auf das Erfassungssystem des CAS-Systems abgestimmt zu sein, es können dann beliebig ausgestaltete Instrumente an den Adapter angekoppelt werden.

Dies erhöht erheblich die Flexibilität bei derartigen Operationen und vereinfacht zugleich die Handhabung des CAS-Systems mit dem entsprechenden Instrumentarium.

In einer weiteren Ausgestaltung der Erfindung ist der Adapter so ausgebildet, daß die beiden Anschlußkupplungen komplementär zueinander sind.

Diese Maßnahme hat nun den erheblichen Vorteil, daß keine baulichen Veränderungen an den Komponenten, bspw. an einer Kamera, durchgeführt werden müssen, die üblicherweise direkt an das Instrument, bspw. an ein Endoskop, angeschlossen werden.

Damit ist es auch möglich, bereits bestehende bzw. standardisierte Bauelemente nach wie vor einzusetzen, es muß lediglich der erfindungsgemäße Adapter zwischengeschaltet werden. Dies trägt nicht nur zur Vereinfachung des CAS-Systems bei, sondern ist äußerst kostengünstig.

In einer weiteren Ausgestaltung der Erfindung weist der Adapter an seiner ersten Seite eine Anschlußkupplung für die Kopplung an eine Okularmuschel auf.

In dieser Ausgestaltung ist der Adapter dazu geeignet, für das am häufigsten bei endoskopischen Eingriffen eingesetzte Instrument, nämlich ein Endoskop, einfach angekoppelt zu werden.

Da Endoskope unterschiedlicher Bauart eines Herstellers oder auch mehrerer Hersteller meist standardisierte Okularmuscheln aufweisen, kann der Adapter einfach an bestehende gängige Endoskopsysteme angekoppelt werden. Eine Okularmuschel bietet relativ große, etwa ringförmige Koppelflächen, so daß der Adapter besonders einfach und fest sitzend anbringbar ist, so daß eine besonders exakte Positionserfassung bei Kopplung mit unterschiedlichen Bauarten von Endoskopen möglich ist.

In einer weiteren Ausgestaltung der Erfindung weist der Adapter an seiner zweiten Seite eine Anschlußkupplung in Form einer Endoskopokularmuschel auf.

Diese Maßnahme hat nun den erheblichen Vorteil, daß der Adapter an dieser zweiten Seite wie die zuvor erwähnten standardisierten Okularmuscheln ausgebildet ist, somit an die entsprechenden standardisierten Koppelstellen von Kameras einfach angekoppelt werden kann. Auch hier erlaubt wieder die Okularmuschelgeometrie eine relativ große und geometrisch günstige, nämlich die kreisförmige Anlagefläche, so daß auch auf dieser Seite eine besonders einfache, rasch durchzuführende und exakt sitzende Verbindung mit den weiteren Bauelementen hergestellt werden kann, so daß insgesamt gesehen der Adapter und somit die Sendereinheit exakt positioniert in das endoskopische System integriert ist.

Es sind auch Ausgestaltungen möglich, bei denen die eigentliche Okularmuschel gegenüber den Standard-Okularmuscheln stark verkleinert ausgeführt ist, wie dies bei reiner Video-Endoskopie üblich ist.

In einer weiteren Ausgestaltung der Erfindung ist der Adapter derart ausgebildet, daß zwischen den Anschlußkupplungen, über die der Adapter mit den beiden Komponenten des endoskopischen Systems koppelbar ist, ein Bild übertragbar ist.

Diese Maßnahme hat nun den erheblichen Vorteil, daß durch die Zwischenschaltung des Adapters eine Bildübertragung, wenn das Instrument ein Endoskop ist, möglich ist und diese nicht nachteilig beeinflußt oder gestört wird. Der Adapter erfüllt also noch eine weitere Funktion, nämlich er dient auch der ungestörten und exakten Bildübertragung.

In vorteilhafter Weise können weitere optische Elemente, insbesondere abbildende Elemente oder Filter, im Adapter angeordnet sein.

In einer weiteren Ausgestaltung der Erfindung ist zwischen den Anschlußkupplungen des Adapters ein optisches Fenster angeordnet, so daß die eine Koppelstelle hermetisch von der anderen Koppelstelle getrennt ist.

Diese Maßnahme hat nun den erheblichen Vorteil, daß der Adapter über das optische Fenster als Trennstelle bspw. zwischen dem sterilen Instrument und der nicht-sterilen Kamera dient, daß zugleich sichergestellt ist, daß ein Bild übertragen werden kann, wenn nämlich das Instrument ein Endoskop ist, und es ist sichergestellt, daß auch trotz mehrerer Wechselvorgänge des Instruments während einer Operation die beiden Komponenten, die an den Adapter angekoppelt sind, hermetisch voneinander getrennt sind, so daß keine Kontaminationen von der einen Komponente zur anderen übertragen werden können. Dies trägt neben der einfachen Handhabung auch noch zur hygienisch sicheren Handhabung des endoskopischen Systems bei.

In einer weiteren Ausgestaltung der Erfindung ist die Anschlußkupplung zum Koppeln des in seiner Funktion zu erfassenden Instruments selbstzentrierend ausgebildet.

Diese Maßnahme hat den Vorteil, daß der Operateur beim Wechseln des Instruments keine besonders hohe Aufmerksamkeit dem Wechsel- und Ankoppelvorgang zuwenden muß, sondern daß lediglich das Instrument an die entsprechende Koppelstelle des Adapters angelegt und bspw. eingedrückt oder eingeschoben werden muß, die Kupplung selbst sorgt dann für die Zentrierung. Dies ist insbesondere bei einer Bildübertragung günstig, da dann die optische Achse des Instruments exakt mit der optischen Achse des Adapters übereinstimmt.

Die durch die maßlichen Toleranzen der Anschlußkupplungen verursachten Positionsfehler sind nach dem Kupplungsvorgang gering.

Dies hat den vorteil, daß der Positionserfassungsfehler aufgrund von Toleranzen äußerst gering ist. Insbesondere bei den eingangs erwähnten Geometrien, also den runden okularmuschelartigen Kopplungsstellen, können exakt sitzende passende Verbindungen ohne großes radiales und axiales Spiel bewerkstelligt werden, wobei trotzdem sichergestellt ist, daß leicht gängige und einfach zu handhabende Kopplungsvorgänge durchgeführt werden können.

Insbesondere sind die Positionsfehler der Anschlußkupplungen reproduzierbar.

Dies hat nun den Vorteil, daß aufgrund dieser reproduzierbaren Abweichungen entsprechende rechnergesteuerte Aus- bzw. Abgleichmaßnahmen ergriffen werden können, die diese Abweichungen in der gesamten Meßgenauigkeit des CAS-Systems berücksichtigen.

In einer weiteren Ausgestaltung der Erfindung ist die Anschlußkupplung zum Koppeln an das eine Instrument derart ausgebildet, daß der Adapter nur in einer bestimmten Relativstellung zum Instrument mit diesem koppelbar und/oder verriegelbar ist.

Bei manchen Instrumenten, bspw. bei Endoskopen mit einem Seitenblick, verläuft die distale Stirnseite nicht exakt rechtwinklig zur Mittellängsachse des Endoskopschaftes, sondern schräg dazu. Das heißt, es gibt Bildeintrittsstellen, die sich näher oder weiter entfernt vom proximalen Ende des Endoskops befinden. In diesem Fall ist es dann gewünscht, das Endoskop in einer einzigen ganz bestimmten Relativdrehstellung zum Adapter bzw. zu der an diesem angebrachten Kamera zu koppeln, was durch entsprechende Orientierungsmerkmale an dem Adapter einfach zu bewerkstelligen ist.

In einer weiteren Ausgestaltung der Erfindung ist die Sendereinheit derart am Adapter angebracht, daß dessen Sender sich in einer Richtung quer zu einer Verbindungslinie zwischen den Anschlußkupplungen erstrecken.

Diese Maßnahme hat nun den Vorteil, daß alle Sender etwa denselben Abstand zur Spitze des Instrumentes besitzen, so daß systemimmanente Fehler, die von der Länge des Abstandes beeinflußt werden, etwa gleich, somit auch wieder rechnerisch zu unterdrücken bzw. zu eliminieren sind. Da die Lokalisationsfehler mit zunehmendem Abstand zwischen der zu lokalisierenden Position, also bspw. der Spitze des Instruments, und den Sendern zunehmen, ist die Anordnung der Sendereinheit quer am Adapter, der wiederum an das proximale Ende des Instruments ansetzbar ist, dahingehend eine gute Lösung, daß dieser Abstand nicht zu groß ist, gleichzeitig aber die Handhabungsfreiheit am Instrument durch den Adapter mit dessen Sendereinheit aber nicht beeinträchtigt ist, so daß bspw. die gesamte Länge des Endoskopschafts durch den Adapter ungehindert bleibt. Diese Ausgestaltung ist ergonomisch und platzsparend.

In einer weiteren Ausgestaltung der Erfindung ist die Sendereinheit etwa stabförmig ausgebildet und erstreckt sich quer zur optischen Achse des Adapters, wobei sich die optische Achse längs einer Verbindungslinie zwischen den Anschlußkupplungen erstreckt.

Diese Maßnahme hat den Vorteil, daß die Sendereinheit als kompaktes Bauelement ausgebildet ist, das die Handhabbarkeit des Adapters erleichtert. Diese Ausgestaltung ist besonders ergonomisch und, was die Baulänge betrifft, besonders günstig.

In einer weiteren Ausgestaltung der Erfindung ist die Sendereinheit derart angeordnet, daß deren Sender sich längs einer Verbindungslinie zwischen den Anschlußkupplungen erstrecken.

Diese Maßnahme hat den Vorteil, daß die Basislänge für die Bestimmung der Spitze des Instrumentes relativ lang ist, somit Meßfehler gering sind. Die Genauigkeit der Erfassung der Drehstellung um die Instrumentenlängsachse ist zwar dann geringer, die Drehstellung als solche ist aber oft weniger genau zu bestimmen. Eine Verteilung längs der Instrumentenachse führt zu einer schlanken und die Sicht längs der Längsachse wenig behindernden Bauweise.

In einer weiteren Ausgestaltung der Erfindung sind die Sender der Sendereinheit flächig verteilt angeordnet.

Diese Maßnahme hat den Vorteil, daß die Genauigkeit der Positionserfassung höchstmöglich ist.

In einer weiteren Ausgestaltung der Erfindung weist die Sendereinheit in an sich bekannter Weise Sender auf, die Ultraschall-, IR- oder andere elektromagnetische Strahlung aussenden.

Diese Maßnahme hat den Vorteil, daß eine berührungslose Erfassung der Position über die von den Sendern abgegebenen Strahlungen bzw. bspw. Felder über Kameras oder andere geeignete Sensoren im Operationssaal erfolgen kann.

In einer weiteren Ausgestaltung der Erfindung weist die Sendereinheit Sender auf, die durch Strahlung aktivierbare Sender aufweist.

Bei aktiv strahlenden Sendern müssen entweder Energiequellen in Form von Batterien oder Kabel zur Verbindung mit abseits liegenden Energiequellen vorgesehen sein, was störend ist. Durch Strahlung aktivierbare Sender, die bspw. in Art von Transpondern aufgebaut sind, brauchen ebenfalls keine eigene Energieversorgung.

Es versteht sich, daß die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine perspektivische Ansicht eines Adapters, wobei eine Koppelstelle ersichtlich ist, die zur Kopplung mit einer Kamera dient,
- Fig. 2: eine weitere perspektivische Ansicht des Adapters von Fig. 1, wobei die der zuvor erwähnten Koppelstelle gegenüberliegende Koppelstelle zum Koppeln mit einem Instrument ersichtlich ist,
- Fig. 3: einen Schnitt längs der Linie III-III in Fig. 1,
- Fig. 4: eine teilweise geschnittene ausschnittsweise Darstellung von zwei Bauelementen, die gegenüberliegend an den Adapter von Fig. 1 bis 3 angekoppelt werden sollen, nämlich an einer Seite ein Endoskop und an der gegenüberliegenden Seite eine Kamera, und
- Fig. 5: stark schematisiert ein CAS-System zusammen mit einem endoskopischen Operationssystem, bei dem, zwischen den beiden Bauelementen Endoskop und Kamera, der Adapter von Fig. 1 bis 4 angeordnet ist.

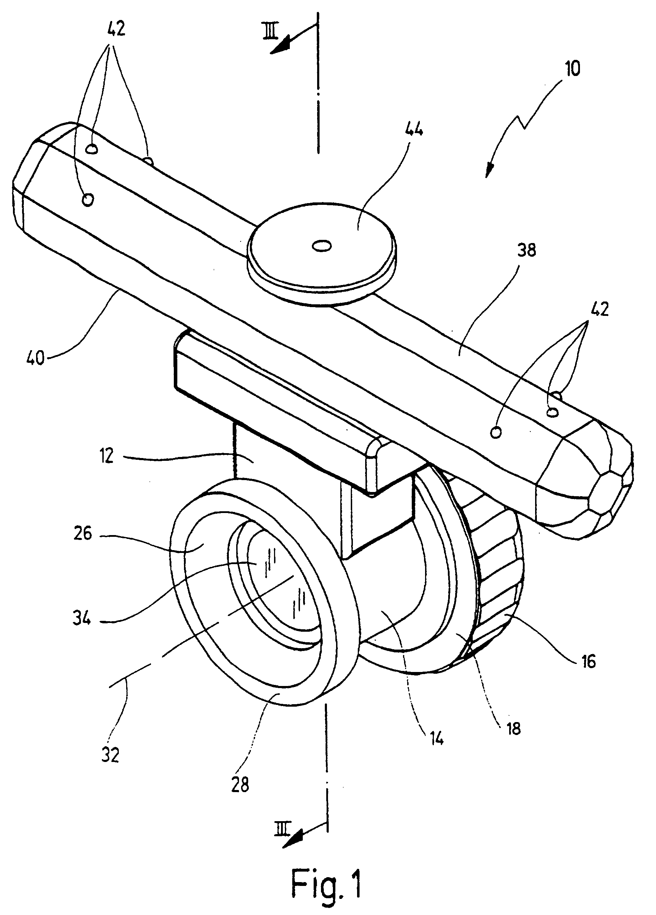

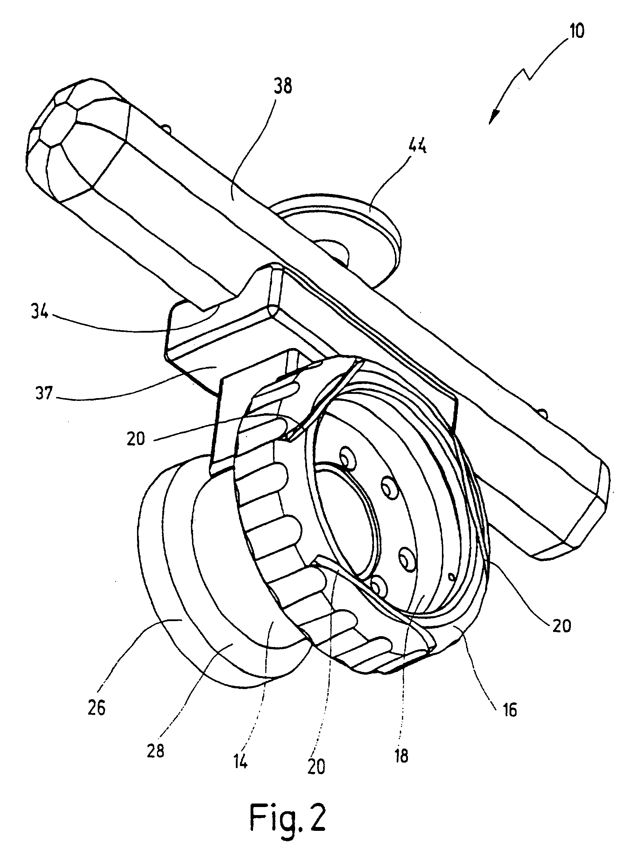

Eine in den Fig. 1 bis 3 dargestellte Vorrichtung mit einer Sendereinheit, über die die Position eines medizinischen Instruments im Rahmen eines CAS-Systems erfaßbar ist, ist in ihrer Gesamtheit mit der Bezugsziffer 10 versehen.

Die Vorrichtung 10 ist als Adapter 12 aufgebaut, der einen mittigen hohlzylindrischen Körper 14 aufweist. An einem Ende des hohlzylindrischen Körpers ist eine Anschlußkupplung 16 vorgesehen.

Die Anschlußkupplung 16 ist als mit hier nicht näher bezeichneten Griffmulden versehener Ring 18 ausgebildet.

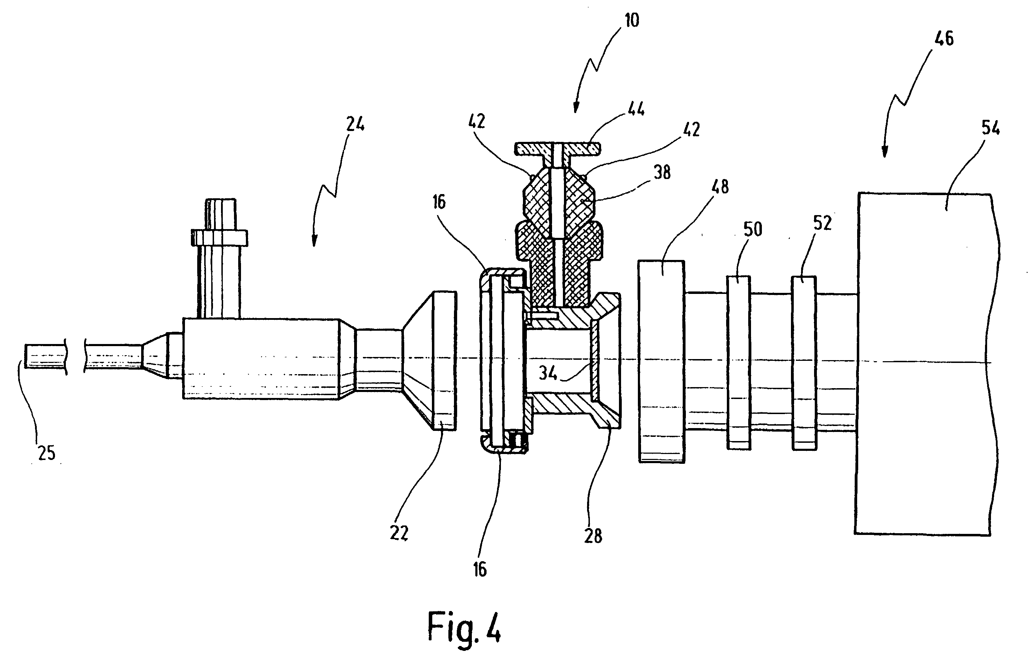

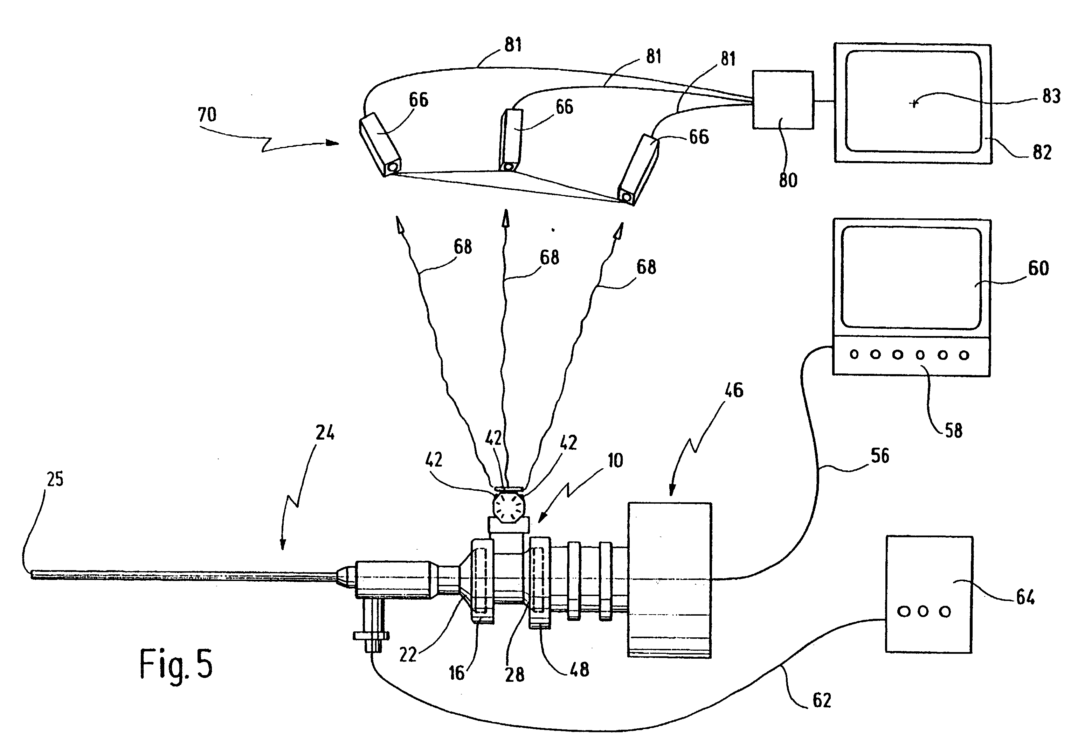

In den Ring sind drei umfänglich gleichmäßig versetzte Einschnitte 20 längs Sekanten des den Ring umgebenden Hüllkreises eingeschnitten. Durch diese Einschnitte entsteht eine gewisse Flexibilität, so daß in den Ring 18 eine Okularmuschel 22 eines Endoskops 24 eingeschoben werden kann, wie das insbesondere aus den Darstellungen von Fig. 4 und 5 ersichtlich ist.

Die Anschlußkupplung 16 dient also dazu, den Adapter 12 an das proximale Ende eines Endoskops 24, also an die umfängliche Ringfläche, dessen Okularmuschel 22 anzukoppeln.

Ist die Okularmuschel 22 eingeschoben, verursachen die umfänglich gleichmäßig verteilten Einschnitte 20 bzw. die dadurch entstehenden beweglichen Teile des Ringes 18 eine exakte Zentrierung der in die Anschlußkupplung 16 eingeschobenen Okularmuschel 22.

Der hohlzylindrische Körper 14 des Adapters 12 weist an dem der Anschlußkupplung 16 gegenüberliegenden Ende eine weitere Anschlußkupplung 26 auf, die die Form einer Okularmuschel 28 aufweist.

Die Form der Okularmuschel 28 entspricht dabei der der Okularmuschel 22, die in die Anschlußkupplung 16 eingeschoben werden kann.

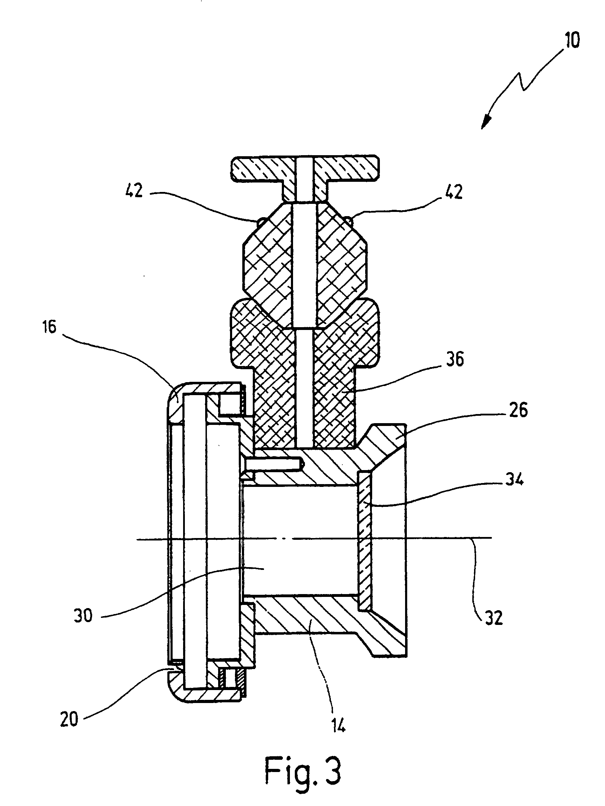

Wie aus der Schnittdarstellung von Fig. 3 zu erkennen, ist im Innern des Adapters ein durchgehender optischer Pfad 30 vorhanden, dessen optische Achse 32 zugleich die Mittellängsachse des hohlzylindrischen Körpers 14 darstellt. Im Bereich der Anschlußkupplung 26 ist ein optisches Fenster 34 eingesetzt, das den Pfad 30 hermetisch trennt.

Anders ausgedrückt, es ist nicht möglich, daß Kontaminationen, die bspw. von einer Systemkomponente bspw. der endoskopischen Kamera 46, an die Anschlußkupplung 26 angeschlossen sind, über den inneren Pfad 30 an das an die andere Anschlußkupplung 16 angekoppelte Instrument, bspw. das Endoskop 24, gelangen können. Dies gilt selbstverständlich auch für den umgekehrten Fall.

Trotz der hermetischen Trennung durch das optische Fenster 34 ist eine Bildübertragung nach wie vor möglich.

An der Außenseite des hohlzylindrischen Körpers 14 des Adapters 12 ist radial abstehend ein Ansatz 36 angebracht, dessen äußeres Ende mit einer Ausnehmung 37 versehen ist, in der eine Sendereinheit 38 eingelegt ist.

Die Sendereinheit 38 ist als Stab 40 ausgebildet, dessen Längsachse sich quer bzw. senkrecht zur optischen Achse 32 erstreckt.

In einer weiteren, hier nicht dargestellten Ausführung erstreckt sich der Stab 40, um 90° gedreht, in Richtung der optischen Achse 32.

Auf dem Stab 40 sind im Bereich dessen gegenüberliegenden Endes jeweils drei Sender 42 flächig verteilt angeordnet, und zwar derart, daß jeweils drei Sender 42 an den Ecken eines Dreiecks liegen. Der Sinn und Zweck dieser Anordnung wird später in Zusammenhang mit Fig. 5 mit der Arbeitsweise eines CAS-Systems beschrieben.

Eine Schraube 44 dient zur Befestigung bzw. zum Wechseln der Sendereinheit 38 am Ansatz 36. Durch diese Verbindung ist die Relativlage zwischen Sendereinheit 38 und Adapter 12 eindeutig und lagefest.

Wie bereits zuvor erwähnt, dient die Anschlußkupplung 16 dazu, um ein Endoskop 24 anzukoppeln, wie das aus der Darstellung von Fig. 4 ersichtlich ist.

Die Anschlußkupplung 26 dient dazu, um mit einer Kamera 46 gekoppelt zu werden.

Die Kamera 46 weist dabei eine Kupplung 48 auf, die über die als Okularmuschel 28 ausgebildete Anschlußkupplung 26 des Adapters 12 geschoben werden kann und über an sich bekannte Elemente verriegelt werden kann.

Die Kupplung 48 ist als Standardkupplung ausgebildet, und dient üblicherweise dazu, die Kamera 46 direkt an eine Okularmuschel 22 eines Endoskops 24 zu koppeln.

Daher ist die Anschlußkupplung 26 als entsprechende Okularmuschel 28 ausgebildet.

Die Kamera 46 weist ferner einen Fokusring 50 und einen Zoom 52 auf, wobei diese Elemente vom eigentlichen Kamerakörper 54 vorstehen.

Zum Verbinden des Adapters 12 mit den beiden Komponenten Endoskop 24 und Kamera 46 werden diese, wie zuvor beschrieben, miteinander verkoppelt.

In Fig. 5 ist dieser Zustand dargestellt, d.h. die Okularmuschel 22 des Endoskops 24 ist in die Anschlußkupplung 16 eingeschoben, die Kupplung 48 der Kamera 46 ist über die als Okularmuschel 28 ausgebildete Anschlußkupplung 26 geschoben.

Dadurch sitzt der Adapter 12 lagedefiniert und fest zwischen diesen beiden Bauelementen, nämlich Endoskop 24 und Kamera 46.

Die Kamera 46 ist über ein Kabel 56 mit einem Video-Prozessor 58 und dieser wiederum mit einem Endoskop-Monitor 60 verbunden. Der Monitor 60 zeigt das durch das Endoskop 24 ersichtliche Bild. Das Endoskop 24 ist über ein Lichtleiterkabel 62 mit einer Lichtquelle 64 verbunden. Dieser Zusammenbau stellt ein endoskopisches System dar.

In dem Raum, in dem der Zusammenbau aus Adapter 12, Endoskop 24 und Kamera 46 eingesetzt werden soll, bspw. in einem Operationssaal, sind zumindest zwei, bevorzugt drei IR-Kameras 66 angeordnet, die die von den Sendern 42 abgestrahlte IR-Strahlung 68 erfassen.

Da, wie zuvor erwähnt, die Sender 42 an den Spitzen eines Dreiecks angeordnet sind, somit eine ganz bestimmte Ebene definieren, ist die Absolutposition des zuvor erwähnten Zusammenbaus über die IR-Kameras 66 eindeutig zu erfassen. Diese Daten werden dann einem CAS-Rechner 80, der über Leitungen 81 mit den IR-Kameras 66 verbunden ist, zugeführt und dort verarbeitet, und zwar zusammen mit Daten über die Lage der Spitze 25 des Endoskops 24 relativ zur Sendereinheit 38. Damit ist die Lage der Spitze 25 des Endoskops 24 eindeutig zu bestimmen.

Entsprechend wurde durch eine Referenzmessung die Lage eines Patienten bestimmt und auch diese Daten dem CAS-Rechner 80 zugeführt. Der CAS-Rechner 80 ist ein Bildverarbeitungssystem, in dem auch die anatomischen Daten des Patienten in Form von CToder MR-Bildern vorhanden sind.

Dadurch ist es nun möglich, auf dem CAS-Monitor 82 die Lage der Spitze 25 des Endoskops in Bezug zu den CT- oder MR-Daten darzustellen, bspw. in Form einer Markierung 83, so daß sich der Operateur anhand des Bildes auf dem CAS-Monitor 82 genau orientieren kann.

Endoskop-Monitor 60 und CAS-Monitor 82 können auch miteinander identisch sein, wobei durch Umschalten oder Aufteilung des Bildes das Endoskop- und das CAS-Bild abwechselnd oder gleichzeitig dargestellt werden können.

Durch die exponierte Lage der Sendereinheit 38 können laufend Positionserfassungen durchgeführt werden, so daß die eingangs erwähnte Technik der Computer Assisted Surgery durchgeführt werden kann.

Das in Fig. 5 nur stark schematisch dargestellte System ist ein sogenanntes CAS (Computer Assisted Surgery)-System 70.

In dem zuvor beschriebenen Ausführungsbeispiel war im Adapter 12 ein optisches Fenster 34 enthalten.

Es sind operative Einsätze denkbar, bei denen der Adapter 12 nur an die Okularmuschel 22 des Endoskops 24 angekoppelt wird, und keine Kamera 46 angekoppelt wird, sondern das Operationsfeld visuell durch das Endoskop bzw. durch den angekoppelten Adapter 12 hindurch beobachtet wird. In diesem Fall ist dann das optische Fenster 34 nicht notwendig. Ist das Endoskop so ausgebildet, daß weitere Kanäle für Instrumente vorgesehen sind, können diese Instrumente dann durch den hohlzylindrischen Körper 14 des Adapters 12 hindurch in das Endoskop eingeschoben werden und damit entsprechende Manipulationen durchgeführt werden.

Da zuvor das Endoskop 24 aber an Ort und Stelle gebracht werden muß, ist dieser Vorgang selbstverständlich wie zuvor beschrieben im Rahmen eines CAS-Systems durchzuführen, das die Lage des Zusammenbaus aus Endoskop 24 und Adapter 12 bzw. die Lage dessen Sender 42 bestimmt.

Chirurgische Instrumente können dann, wenn ihr Endstück okularmuschelförmig ausgebildet ist, ebenfalls mit dem erfindungsgemäßen Adapter gekoppelt werden. Dies gilt bspw. dann, wenn ein Trokar mit einem Obturator ohne Sichtkontrolle an den Operationsort geführt wird. Das CAS-System ermöglicht dann die laufende Überwachung der erreichten Position. In diesem Fall bleiben die optische Durchgängigkeit und die Möglichkeit der proximalseitigen Kopplung des Adapters ungenutzt.

Zuvor wurde beschrieben, daß bspw. die exakte Lage der Spitze 25 des Endoskopes 24 bestimmt werden soll. Es ist auch möglich, eine bestimmte Stelle abseits der Spitze 25, bspw. eine Stelle wenige Zentimeter vor der Spitze, genau zu erfassen. Diese Stelle ist deswegen von Interesse, da bei einem endoskopisch erfaßten operativen Eingriff nicht die Stelle unmittelbar an der Spitze von vorrangigem Interesse ist, sondern eine Stelle einige Zentimeter vor der Spitze, nämlich eine Stelle, an der bspw. der eigentliche operative Eingriff durchgeführt werden soll. Diese Stelle kann für den Operateur dadurch sichtbar gemacht werden, daß über die Lichtleiter im Endoskop, bspw. diametral gegenüberliegend, zwei Laserstrahlen zur Spitze 25 geführt werden. Der Austritt ist dann so, daß sich diese beiden Strahlen wenige Zentimeter vor der Spitze kreuzen und für den Operateur einen deutlich ersichtlichen Lichtpunkt ergeben, der genau der Stelle entspricht, die eigentlich beobachtet werden soll. Das CAS-System kann nun diesen Ort des "verlängerten" Endoskopes in den drei D-Datensatz einblenden, und der Operateur kann den Schnittpunkt der beiden Laserstrahlen in seinem Blickfeld auf das interessierende Gewebe richten.

## Patentansprüche

1. Adapter, zum Verbinden zumindest zweier medizinischer Instrumente, mit einer Anschlußkupplung (16) an einer ersten Seite, über die der Adapter (12) an eine vorbestimmte Koppelstelle an dem einen medizinischen Instrument (24) lösbar, jedoch mechanisch festsitzend einfach ankoppelbar ist, und wobei der Adapter (12) zumindest eine weitere Anschlußkupplung (26) aufweist, über die er mit einem weiteren medizinischen Instrument koppelbar ist,
**dadurch gekennzeichnet,**
**daß** der Adapter (12) eine Sendereinheit (38) aufweist, über die die Position des einen, an den Adapter (12) angekoppelten medizinischen Instrumentes (24) mit Hilfe eines CAS-Systems (70) erfaßbar ist, wobei die Sendereinheit (38) lagefest zum Adapter (12) ist, und wobei die Sendereinheit (38) mehrere auf der Sendereinheit (38) verteilt angeordnete Sender (42) aufweist, deren Position vom CAS-System (70) erfaßbar ist.

2. Adapter nach Anspruch 1, **dadurch gekennzeichnet, daß** die beiden Anschlußkupplungen (16, 26) komplementär zueinander sind.

3. Adapter nach Anspruch 2, **dadurch gekennzeichnet, daß** dieser an seiner ersten Seite eine Anschlußkupplung (16) für die Kopplung an eine Okularmuschel (22) aufweist.

4. Adapter nach Anspruch 3, **dadurch gekennzeichnet, daß** dieser an seiner zweiten Seite eine Anschlußkupplung (26) in Form einer Endoskopokularmuschel (28) aufweist.

5. Adapter nach einem der Ansprüche 1 bis 4, **dadurch gekennzeichnet, daß** dieser derart ausgebildet ist, daß zwischen den Anschlußkupplungen (16, 26), über die der Adapter (12) mit den beiden Komponenten (24, 46) des endoskopischen Systems koppelbar ist, ein Bild übertragbar ist.

6. Adapter nach Anspruch 5, **dadurch gekennzeichnet, daß** zwischen den Anschlußkupplungen (16, 26) ein optisches Fenster (34) angeordnet ist, so daß die eine Koppelstelle hermetisch von der anderen Koppelstelle getrennt ist.

7. Adapter nach Anspruch 5 oder 6, **dadurch gekennzeichnet, daß** zwischen den Anschlußkupplungen (16, 26) weitere optische Elemente, insbesondere abbildende Elemente oder Filter, angeordnet sind.

8. Adapter nach einem der Ansprüche 1 bis 7, **dadurch gekennzeichnet, daß** die Anschlußkupplung (16) zum Koppeln des in seiner Position zu erfassenden Instruments selbstzentrierend ausgebildet ist.

9. Adapter nach einem der Ansprüche 1 bis 8, **dadurch gekennzeichnet, daß** die Anschlußkupplung (16) zum Koppeln an das eine Instrument (24) derart ausgebildet ist, daß der Adapter nur in einer bestimmten Relativstellung zum Instrument (24) mit diesem koppelbar und/oder verriegelbar ist.

10. Adapter nach einem der Ansprüche 1 bis 9, **dadurch gekennzeichnet, daß** die Sendereinheit (38) derart am Adapter (12) angebracht ist, daß dessen Sender (42) sich in einer Richtung quer zu einer Verbindungslinie zwischen den Anschlußkupplungen (16, 26) erstrecken.

11. Adapter nach Anspruch 10, **dadurch gekennzeichnet, daß** die Sendereinheit (38) etwa stabförmig ausgebildet ist und sich quer zu einer optischen Achse (32) des Adapters (12) erstreckt, wobei die optische Achse (32) sich längs einer Verbindungslinie zwischen den Anschlußkupplungen (16, 26) erstreckt.

12. Adapter nach einem der Ansprüche 1 bis 9, **dadurch gekennzeichnet, daß** die Sendereinheit (38) derart angeordnet ist, daß sich deren Sender längs einer Verbindungslinie zwischen den Anschlußkupplungen (16, 26) erstrecken.

13. Adapter nach einem der Ansprüche 1 bis 12, **dadurch gekennzeichnet, daß** die Sender (42) der Sendereinheit (38) flächig verteilt angeordnet sind.

14. Adapter nach einem der Ansprüche 1 bis 13, **dadurch gekennzeichnet, daß** die Sendereinheit (38) Sender (42) aufweist, die Ultraschall-, IR- oder andere elektromagnetische Strahlung aussenden.

15. Adapter nach einem der Ansprüche 1 bis 13, **dadurch gekennzeichnet, daß** die Sendereinheit Sender aufweist, die durch Strahlung aktivierbare Sender gebildet sind.

## Claims

1. Adapter for connecting at least two medical instruments, comprising a receptor coupling (16) on a first side with which the adapter (12) can be releasably but mechanically fixed and simply coupled to a predetermined coupling location on a medical instrument (24) and said adapter (12) comprising at least a further receptor coupling (26), with which it can be coupled to a further medical instrument,
**characterized in that**
the adapter (12) is provided with a transmitter unit (38) via which the position of the one medical instrument (24) coupled to the adapter (12) can be detected with the aid of a CAS system, said transmitter unit (38) being arranged in a fixed position on the adapter (12),
and **in that**
several transmitters (42) are arranged and distributed on the transmitter unit (42) which positions can be detected by the CAS system (70).

2. Adapter of claim 1, **characterized in that** the two receptor couplings (16, 26) are complementary to one another.

3. Adapter of claim 2, **characterized in that** it comprises at its first side a receptor coupling (16) for coupling to an ocular cup (22).

4. Adapter of claim 3, **characterized in that** it comprises a receptor coupling (26) on its second side in the form of an endoscopic ocular cup (28).

5. Adapter of anyone of claims 1 through 4, **characterized in that** it is configured such that an image can be transmitted between the receptor couplings (16, 26) via which the adapter (12) is coupled with two components (24, 46) of the endoscopic system.

6. Adapter of claim 5, **characterized in that** an optical window (34) is arranged between the two receptor couplings (16, 26) so that the one coupling location is hermetically separated from the other coupling location.

7. Adapter of claims 5 or 6, **characterized in that** further optical elements, in particular imagining elements or filters are arranged between the receptor couplings (16, 26).

8. Adapter of anyone of claims 1 through 7, **characterized in that** the receptor coupling (16) for coupling the instrument whose position is to be detected is formed to be self-centering.

9. Adapter of anyone of claims 1 through 8, **characterized in that** the receptor coupling (16) for coupling to the instrument (24) is configured such that the adapter can only be coupled and/or locked in a predetermined relative position to the instrument (24).

10. Adapter of anyone of claims 1 through 9, **characterized in that** the transmitter unit (38) is attached to the adapter (12) such that its transmitters (42) are aligned in a direction transverse to a connecting line between the receptor couplings (16, 26).

11. Adapter of claim 10, **characterized in that** the transmitter unit (38) is approximately bar-shaped and extends transversely to the optical axis (32) of the adapter (12), said optical axis (32) extending along a connecting line between the receptor couplings (16, 26).

12. Adapter according to anyone of claims 1 through 9, **characterized in that** the transmitter unit (38) is arranged such that its transmitters extend along the connecting line between the receptor couplings (16, 26).

13. Adapter of anyone of claims 1 through 12, **characterized in that** the transmitters (42) of the transmitter unit (38) are arranged to be distributed over a surface.

14. Adapter of anyone of claims 1 through 13, **characterized in that** the transmitter unit (38) comprises transmitters (42) which transmit ultrasound, IR or other electromagnetic radiation.

15. Adapter of anyone of claims 1 through 13, **characterized in that** the transmitter unit comprises transmitters which are radiation activated transmitters.

## Revendications

1. Adaptateur pour relier au moins deux instruments médicaux, avec une prise de raccordement (16) d'un premier côté, par laquelle l'adaptateur (12) peut être facilement accouplé de facon démontable, mais mécaniquement solidaire, en un emplacement d'accouplement prédéfini sur l'un des instruments médicaux (24), et l'adaptateur (12) comprenant au moins une autre prise de raccordement (26), par laquelle il peut être accouplé à un autre instrument médical,
**caractérisé en ce que**
l'adaptateur (12) comprend une unité émettrice (38), par laquelle la position de l'un des instruments médicaux (24) accouplé à l'adaptateur (12) peut être détectée à l'aide d'un système CAS (70), l'unité émettrice (38) ayant une position fixe par rapport à l'adaptateur (12), et l'unité émettrice (38) comprenant plusieurs émetteurs (42) répartis sur l'unité émettrice (38), dont la position peut être détectée par le système CAS (70).

2. Adaptateur selon la revendication 1, **caractérisé en ce que** les deux prises de raccordement (16, 26) sont complémentaires l'une de l'autre.

3. Adaptateur selon la revendication 2, **caractérisé en ce que** ce dernier comporte sur son premier côté une prise de raccordement (16) pour l'accouplement sur une bonnette d'oculaire (22).

4. Adaptateur selon la revendication 3, **caractérisé en ce que** ce dernier comporte sur son deuxième côté une prise de raccordement (26) pour une bonnette d'oculaire d'un endoscope (28).

5. Adaptateur selon l'une des revendications 1 à 4, **caractérisé en ce que** ce dernier est configuré de telle sorte qu'une image peut être transmise entre les prises de raccordement (16, 26) par lesquelles l'adaptateur (12) peut être accouplé aux deux composants (24, 46) du système endoscopique.

6. Adaptateur selon la revendication 5, **caractérisé en ce qu'**entre les prises de raccordement (16, 26) est disposée une fenêtre optique (34), de telle sorte que l'un des emplacements d'accouplement est séparé hermétiquement de l'autre emplacement d'accouplement.

7. Adaptateur selon la revendication 5 ou 6, **caractérisé en ce qu'**entre les prises de raccordement (16, 26) sont disposés d'autres éléments optiques, en particulier des éléments de reproduction d'image ou des filtres.

8. Adaptateur selon l'une des revendications 1 à 7, **caractérisé en ce que** la prise de raccordement (16) pour l'accouplement de l'instrument devant être détecté dans sa position est configurée autocentrante.

9. Adaptateur selon l'une des revendications 1 à 8, **caractérisé en ce que** la prise de raccordement (16) pour l'accouplement à l'un des instruments (24) est configurée de telle sorte que l'adaptateur ne peut être accouplé et / ou verrouillé sur l'instrument (24) que dans une position relative définie par rapport celui-ci.

10. Adaptateur selon l'une des revendications 1 à 9, **caractérisé en ce que** l'unité émettrice (38) est montée de telle sorte sur l'adaptateur (12), que ses émetteurs (42) s'étendent dans une direction transversale par rapport à une ligne reliant les prises de raccordement (16, 26).

11. Adaptateur selon la revendication 10, **caractérisé en ce que** l'unité émettrice (38) est configurée sensiblement en forme de barreau et s'étend transversalement par rapport à un axe optique (32) de l'adaptateur (12), l'axe optique (32) s'étendant le long d'une ligne reliant les prises de raccordement (16, 26).

12. Adaptateur selon l'une des revendications 1 à 9, **caractérisé en ce que** l'unité émettrice (38) est disposée de telle sorte que ses émetteurs s'étendent le long d'une ligne reliant les prises de raccordement (16, 26).

13. Adaptateur selon l'une des revendications 1 à 12, **caractérisé en ce que** les émetteurs (42) de l'unité émettrice (38) sont répartis sur une surface.

14. Adaptateur selon l'une des revendications 1 à 13, **caractérisé en ce que** l'unité émettrice (38) comporte des émetteurs (42) qui émettent des ultrasons, des rayons IR ou d'autres rayonnements électromagnétiques.

15. Adaptateur selon l'une des revendications 1 à 13, **caractérisé en ce que** l'unité émettrice comporte des émetteurs qui sont formés par des émetteurs qui peuvent être activés par rayonnement.
